Europäisches Patentamt

(19) European Patent Office

Office européen des brevets

(11) Publication number: **0 169 055 B1**

# (12) EUROPEAN PATENT SPECIFICATION

(45) Date of publication of patent specification: **14.08.91**

(51) Int. Cl.⁵: **G01N 33/52**, G01N 33/72, G01N 33/84, C12Q 1/60, C12Q 1/54, C12Q 1/62, G01N 33/53

(21) Application number: **85305059.9**

(22) Date of filing: **16.07.85**

(54) **Polymeric single layer analytical element.**

(30) Priority: **17.07.84 US 631677**

(43) Date of publication of application:
**22.01.86 Bulletin 86/04**

(45) Publication of the grant of the patent:
**14.08.91 Bulletin 91/33**

(84) Designated Contracting States:
**BE CH DE FR GB IT LI NL SE**

(56) References cited:
**EP-A- 0 010 615**
**WO-A-79/01081**
**AT-B- 371 606**

(73) Proprietor: **TECHNICON INSTRUMENTS COR-PORATION(a Delaware corporation)**
**511 Benedict Avenue**
**Tarrytown, New York 10591-5097(US)**

(72) Inventor: **Kumar, Anand**
**1 Duelk Avenue**
**Monroe, NY 10950(US)**
Inventor: **Koon-Wah, Leong**
**71 Van Cortlandt Avenue**
**Ossining, NY 10562(US)**

(74) Representative: **Wain, Christopher Paul et al**
**A.A. THORNTON & CO. Northumberland House 303-306 High Holborn**
**London WC1V 7LE(GB)**

## Description

This invention relates generally to the field of fluid analysis by analytical devices or elements.

Reagent compositions and devices incorporating them have been known for many years and used in a wide variety of fields, from pH test papers to in-vitro diagnostic devices for detecting body fluid components and therapeutic agents in body fluids. The reagent compositions and devices are contacted with fluids in which the constituent(s) are to be determined. A detectable change is effected by chemical or other reaction.

The first type of such device which was developed had an irregular; fibrous reagent layer, such as paper. The thickness of the layer was not critical, but needed only to be sufficient to render the reaction layer opaque, e.g. it could be of infinite optical thickness. Analyte concentration was semi-quantitatively determined by a visual comparison of the diffuse reflectance signal with a color block chart (see, for example, U.S. patents nos. 3,012,976, 3,164,934 and 3,485,587, and Walter, Anal. Chem. 55:498A (1983)). The irregularity of these types of elements severely limited the quantitation which could be achieved. Even the introduction of photometric instrumentation has not overcome this fundamental limitation.

The art has evolved such that most "dry chemistry" elements are now monitored by reflectance methods and include a plurality of layers in various laminar configurations. Each of these is a discrete, separate layer which performs one or more, but less than all, of the necessary functions and is bound to the other layers. The one or more reagent layers incorporate a composition which usually includes all the reagents necessary for analysis of a particular body fluid constituent, therapeutic drug or other analyte in order to provide a measurable response. See Walter, et al., supra and also Shirey, Clin. Biochem., 16: 147-155 (1983). In such elements incident light passes through the translucent reagent layer(s) where it is absorbed to an extent dictated by the analyte/reagent composition. This requires that the reagent layer(s) be uniform and of precisely controlled thickness. A smooth, opaque reflective layer is laminated to the opposite side of the reagent layer. This reflects the unabsorbed light back through the reagent layer to a detector.

One of the simplest analytes for determination in conventional chemistry systems has been albumin. In such "dry chemistry" methodologies, even this analyte has required a multilayer element. Wu, U.S. patent no. 4,230,456 discloses a multilayer analytical element for albumin which includes a spreading layer, a subbing layer and a reagent layer. The reagent layer contains a complex of anilinonaphthalene sulfonic acid (ANS) and polyvinyl alcohol (PVA). Sample albumin passes into the spreading layer but not normally into the reagent layer. Nonetheless, it is said that this albumin causes release of the ANS from the complex into the spreading layer. It is also said that, in use, when albumin is quantified by measuring formation of a complex of albumin and detectable species, it is desirable to use a multilayer analytical element having a transparent spreading layer and opaque reagent layer and that any suitable opacifying agent, such as titanium dioxide, can be used. The method described for reading the result is by passing the element through a zone in which suitable spectrophotometry apparatus directs a beam of energy, such as light, through the support and the reagent layer. It is unclear how this would be operative where the detectable species migrates to a separate layer on the opposite side of the opaque reagent layer.

In yet another type of device, reaction layers have been formed of hollow microspheres or beads and the necessary reagents incorporated within their interior. This approach is disclosed inter alia in U.S. patents nos. 3,092,463; 3,926,732; 3,993,451; 4,129,417; 4,356,149; and WO 79/01081. For example, Siddigi, WO 79/01081 discloses a test device made of an inert support covered with a layer of polymer beads which contain reagent and in which the color reaction takes place. By way of further example, Kitajima, et al, U.S. patent no. 4,356,149 discloses a multilayer element in which the reaction layer is of an oil-in-water type dispersion wherein the reagent is contained in hydrophobic particles dispersed in a hydrophilic binder. The multilayer element disclosed also includes a radiation-blocking or light reflection layer between the reagent layer and an aqueous liquid sample-speading layer.

Pierce, et al, U.S. patent nos. 4,258,001 and 4,381,921 (a division of 4,258,001) reflect a further development in the field of multilayer analytical elements which emphasizes a complex three-dimensional lattice structure. These lattice structures are very difficult to manufacture and are commercially not available. Overall, they apply this structure in the type of multilayer elements described above. They also allude to the use of this structure apart from spreading, reflective or other layers. The only two analytical examples of this lattice structure, apart from other layers, are limited to "qualitative" results for glucose determination over a narrow concentration range and application to hemoglobin analysis for which no data in differentiating hemoglobin concentrations are reported. The three-dimensional lattice structure is formed of heat stable, non-swellable organo-polymeric particles and adhesive for these particles. Preferably, reagents are chemically bonded to the polymer units of the particles. Although particle size and shape can vary, they are preferably uniform and spherical. The particles typically comprise at least about 25 weight percent, and preferably 50 weight percent or more, of the organopolymeric material. In many embodiments, these particles are composed entirely, i.e. 100 weight percent, of such organopolymeric material. The remainder of these particles can be composed of other addenda, for example, colorants such as pigments or dyes.

In summary, the earliest reagent-impregnated paper strips have been limited in precision and use in quantitation whereas the quantitation achieved by multilayered elements has been at the cost of increased complexity and susceptibility to fine variation in manufacturing specifications. The encapsulation of reagents has neither reduced this complexity nor reduced the susceptibility of readings to interference. The preparation of elements formed of complex three-dimensional, organo-polymeric lattices does not reduce the complexity of manufacture nor does it impart satisfactory reflectance characteristics to the described layer.

In contrast to the reagent matrices or elements previously described, the present invention provides the combination of a complete reagent system and a reflective component in a single layer which can be read by diffuse reflectance. The adverse influence of sample turbidity can thus be avoided, and also the restrictive requirements of uniformity and precise thickness of the matrix are avoided in that light is not required to pass completely through the reagent in order to be reflected for detection. In addition, the elements can include components which prevent areas of uneven reagent distribution with their risk of erroneous quantitative readings.

Further, the elememts of the invention can provide a previously unachieved broad analyte menu which can be incorporated into such elements, including homogeneous immunoassay reagent systems and conventional clinical chemistry reagent systems, all without the need for predilution of sample before application to the element. The need for predilution of sample in prior art analytical elements has been a major drawback to their suitability for use. The lower scattering co-efficient of the matrix makes possible the higher sensitivity to low concentration analytes as compared to the paper or other matices and spreading layers which characterize prior art devices. The concentration range of analytes extends from very low levels of blood enzymes, hormones and therapeutic drugs to very ·high concentration analytes, such as glucose.

These and other advantages are achieved by a single layer analytical element for determining an analyte in a fluid sample, which element consists of a dimensionally stable, uniformly porous, diffusely reflective single layer formed of a polymeric non-fibrous matix and, dispersed throughout said matrix, at least one analyte-responsive substance which effects a detectable response to the analyte. The invention also includes an analytical device which comprises an analytical element of the invention mounted on an inert carrier.

Preferably, the polymer is a high density polyethylene treated to be hydrophilic. The polymers preferably have omnidirectional, interconnecting pores, the size of which can be accurately controlled between 5 and 500 micrometers. The optical properties can be further modified by blending in pigments, such as titanium dioxide. The analyte-responsive substance can include a first component which is responsive to the analyte to form a reaction product, and a second component responsive to the reaction product to effect a detectable change. The single layer of the element can further incorporate a high molecular weight substance which reduces or minimizes the migration of reagent due to the flow forces resulting from application of liquid sample thereto. This prevents areas of reagent concentration differential from forming, thereby preventing inconsistency of the level of detectable response from area to area on the single layer. Macromolecular substances suitable for use in the elements of the invention include proteins, gums, waxes or, preferably, polymers. The polymer can be, for example, cellulose acetate, polyvinyl chloride, nylon, polyacrylates, starch, polycarbonate, polyvinyl alcohol, polyvinyl acetate, dextran, polyvinyl pyrrolidone, polyurethane, cellulose, cellulose nitrate or cellulose acetate butyrate. For example, each 100 ml of the composition which is used to form the analytical layer can include 1-9 grams (g) polyvinylpyrrolidone. The element is integral in that the single layer is completely effective in the absence of other elements or structures to provide a quantitative detectable response to the presence of the analyte under assay.

The invention also provides a method of determining an analyte in a fluid sample by contacting an element or device of the invention with a sample suspected to contain the analyte and observing any detectable response. Various embodiments, which include or use the element, device and/or method are also contemplated. For example, the test material can be provided as part of a test kit. The kit comprises the packaged combination of one or more elements or devices or containers of them in any of a variety of physical formats.

In order that the invention may be more fully understood, a preferred embodiment thereof will now be described by way of example only with reference to Figure 1 of the accompanying drawings, and Figures 2 to 8 relate to the Examples hereafter, as follows:

FIGURE 1 is a schematic side view of one embodiment of the device of the invention.

FIGURE 2 is a graphical illustration of the data resulting from the experiments described in Example 1.

FIGURE 3 is a graphical illustration of the data resulting from the experiments described in Example 2.

FIGURE 4 is a graphical illustration of the data resulting from the experiments described in Example 3.

FIGURE 5 is a graphical illustration of the data resulting from the experiments described in Example 4.

FIGURE 6 is a graphical illustration of the data resulting from the experiments described in Example 5.

FIGURE 7 is a graphical illustration of the data resulting from the experiments described in Example 6.

FIGURE 8 is a graphical illustration of the data resulting from the experiments described in Example 7.

Specific terms in the following description which refer only to a particular embodiment are exemplary of all of the embodiments unless otherwise indicated

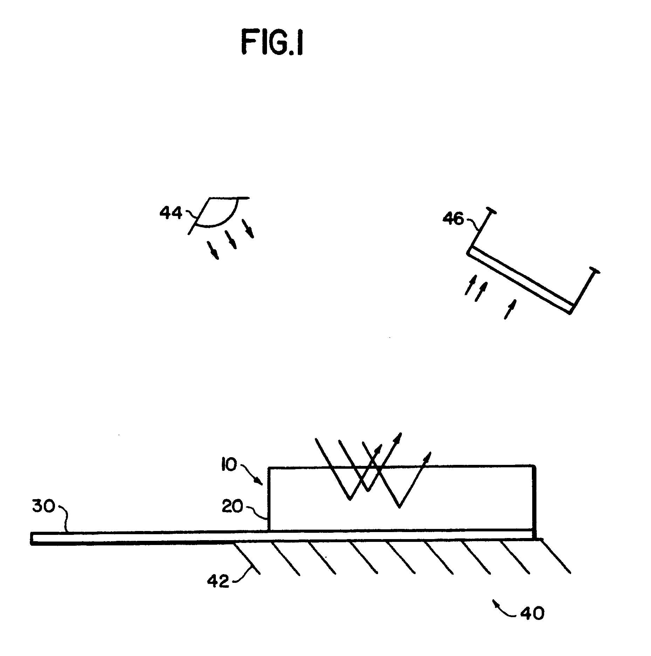

Fig. 1 illustrates a preferred embodiment of the device of the present invention as it can be used with a reflectance instrument. The device 10 has a single analysis layer 20. Layer 20 is comprised of a reflective polymeric matrix having reagent dispersed throughout the matrix. The single layer matrix is usually formed by sintering polymeric particles to impart dimensional stability to the single layer configuration. Device 10 also has a carrier 30 which takes no part in the analysis, and is provided only for ease of handling. In accordance with the present invention even such a carrier is not required, and is usually not included, because of the dimensional stability of the single layer. Liquid sample is applied to device 10 to react with reagent in analysis layer 20. Device 10 is positioned in an instrument 40 on base 42. The instrument has a light source 44 which directs light on and into layer 20. A detector 46 detects the amount of reflected light and the instrument reports this as a function of analyte concentration. The light source 44 and detector 46 can also be positioned to direct incident light and detect reflected light from either side, by the same side to which sample is applied or the opposite side.

· Sample fluids on which tests are performed include biological, physiological, industrial, environmental, and other types of liquids or gases. Of particular interest are biological liquids such as serum, plasma, urine, cerebrospinal fluid, saliva, milk, broth and other culture media and supernatants as well as fractions of any of them. Biological species of interest in gaseous form include ammonia. Physiological fluids of interest include infusion solutions, buffers, preservative or antimicrobial solutions and the like. Industrial liquids include fermentation media and other processing liquids used, for example, in the manufacture of pharmaceuticals, dairy products and malt beverages. Other sources of sample fluid which are tested by conventional methods are contemplated as within the meaning of this term as used and can, likewise, be assayed in accordance with the invention.

The solid, reflective, particles used to prepare the polymeric matrix are optically opaque granular or particulate matter which have no reagent or substantial internal void volume and are chemically inert to the sample, reagents and reaction for which the device is intended. Within this limitation it is desirable to select particles which produce a matrix that is not light absorbing at the detection wavelength and have as high a refractive index as possible, preferably above about 1.6. Preferably, the particles have a diameter of from about 0.1 microns to about 200 microns.

The preferred polymer matrix for the analytical element is porous, uniform omnidirectionally, dimensionally stable, chemically inert, and non-swellable by reagent or analytical sample carriers. It is also optically compatible to reflective measurement such that it has little or no absorptivity in the ultraviolet and visible regions, e.g., 300 nm - 700 nm. The matrix can be of various porosities, pore sizes, thicknesses, shapes and polymer compositions at low costs. Examples of materials which can be used for this particular application are Interflo® Porous Plastic available from Chromex Corp, Brooklyn, New York and Porex® Porous Plastics available from Porex Technologies, Fairburn, Georgia. The use of porous plastics as a matrix for "dry chemical" reactions has not been reported in the literature.

The polymers which are particularly useful in forming the matrix of the analytical element are high-density polyethylene (HDPE), ultra-high molecular weight polyethylene, polypropylene, polyvinylidene fluoride (Kynar®), polytetrafluoroethylene (PTFE), nylon®, polyvinylchloride (PVC), polyesters, polysulfones, and other polymers and polymer blends.

It can be advantageous to use hydrophobic polymer particles to prepare the matrix, and to impart hydrophilic characteristics thereto by incorporating one or more emulsifier or surfactant materials, such as anionic, cationic and non-ionic surfactants in the analytical element. However, non-ionic surfactants are preferred. They can, for example, also enhance coatability of composition formulations and enhance the extent and range of wetting in elements that are not easily wetted by liquid samples in the absence of an aid such as a surfactant. The properties of the polymers can be further modified by blending in such surfactants during the formulation of the polymeric matrix or by surface coating the preformed polymeric matrix with such surfactant. For example, each 100 gram of the composition which is formed into elements can include 0.1 gram of surface-active agents like the fatty acid partial esters of sorbitol anhydrides. Other examples of surfactants are Triton® X-100 (from Rohn and Haas, Philadelphia, PA). Span® 20, Brij® 35 and Tween® 20 (from ICI Americas, Wilmington, DE), Zonyl® FSN (from E.I. duPont de-Nemours, Wilmington, DE), and Pluoronics (from BASF Wyandotte, Parsippany, NJ).

The single layer can also be made to include irregular, reflective inorganic particles. The combination of high refractive index and irregular particle shape enhances the scattering of incident radiation, thereby increasing analytical signal in a diffuse, reflectance colorimetric mode. This is of advantage where analytes of high sample concentration or high extinction coefficient are of interest. Particles which are most preferred include basic lead hydroxide, basic lead carbonate; oxides such as titanium dioxide, zinc oxide and magnesium oxide; and sulfates such as barium sulfate, calcium sulfate and strontium sulfate. Other materials which can be used include silica, calcite, silicates and aluminosilicates, zinc sulfide, diatomaceous earth, clay or mixtures thereof.

The at least one substance which effects a detectable response in the presence of the analyte can be appropriately selected from those reagent compositions known for the selective detection of analytes of interest. In one embodiment it can include a substance which physically changes the sample, such as a cytolytic agent, to release an analyte for detection. For example, where the analyte is hemoglobin the substance can be a cytolytic agent, such as a surfactant. The surfactant can, for example, be coated onto the polymeric matrix and includes polyoxyethylene ethers of aliphatic alcohols such as Brij® surfactants (ICI United States, Wilmington, DE), polyoxyethylene derivatives of sorbitol anhydride fatty acid partial esters such as Tween® surfactants (ICI United States, supra) and alkylaryl polyethers of alcohols, sulfonates, or sulfates such as Triton® surfactants (Rohm & Haas Company, Philadelphia, PA). In another embodiment, the analyte-responsive substance can include a relatively simple chromogenic reagent. One example of this embodiment is the use of bromcresol green in determining albumin. By way of additional example, where the analyte is bilirubin, it can include a diazo compound, such as diazo sulfanilic acid, and a displacing agent, an agent which frees bilirubin from its bound form, such as diphylline, caffeine or dimethylsulfoxide.

Where the analyte is glucose, the composition includes a first and second component which are included in the preparation simultaneously. The first component includes glucose oxidase. For example, the reagent content for each 5-15 ml of 0.2 molar (M) phosphate buffer (pH 7.0), can include 4,500-40,000 International Units (IU) glucose oxidase, 2,000-40,000 IU peroxidase, 250-1,000 milligrams (mg) bovine serum albumin, 250-1,500 mg 4-aminoantipyrine, 100-30,000 mg sodium p-hydroxbenzoate, 5-50 mg disodium EDTA (ethylenediamine tetraacetic acid), 5-20 mg potassium azide and 5-400 mg polyvinylpyrrolidone. The Commission on Enzymes of the International Union of Biochemistry has defined an International Unit (IU) of enzyme activity as 1 micromole (umol) of substrate utilized per minute under specified condition of pH and temperature.

In another preferred embodiment the first component is reactive with the analyte to produce hydrogen peroxide and the second component includes a peroxidatively active substance, such as peroxidase, and a redox indicator. For example, where the analyte is cholesterol, the first component includes cholesterol oxidase and cholesterol ester hydrolase. Where the analyte is uric acid, the first component includes uricase.

The term "peroxidatively active substance" defines the precise chemical activity of the substances contemplated. A peroxidase is an enzyme which will catalyze a reaction wherein hydrogen peroxide oxidizes another substance. The peroxidases are generally proteins which incorporate iron porphyrin moieties. Peroxidase occurs in horseradish, potatoes, fig-tree sap and turnips (plant peroxidase); in milk (lacto peroxidase); and in white blood corpuscles (verdo peroxidase); also it occurs in microorganisms and may be produced by fermentation. Certain synthetic peroxidases, such as those disclosed by Theorell and Maehly in Acta Chem. Scand., 4:422-434 (1950), are also satisfactory for use in $H_2O_2$ detection systems. Less satisfactory are such substances as hemin, methemoglobin, oxyhemoglobin, hemoglobin, hemochromogen, alkaline hematin, hemin derivatives, and certain other compounds which demonstrate peroxidative or peroxidase-like activity, namely, the ability to catalyze the oxidation of another substance by means of hydrogen peroxide and other peroxides. Other substances which are not enzymes but which demonstrate peroxidative activity are for example: iron sulfocyanate, iron tannate, ferrous ferrocyanide, or chromic salt (such as potassium chromic sulfate) absorbed on silica gel.

The redox indicators contemplated include the many which are known to those skilled in the art. These include benzidine, o-toluidine, 3,3',5,5'-tetramethylbenzidine, 2,7-diaminofluorene, 3-methyl-2-benzothiazolinone hydrazone, optionally with its known couplers, 4-aminoantipyrine, optionally with its known couplers and other phenazones and pyrones.

Alternatively the analyte-responsive substance can comprise reagents for a homogeneous specific binding assay system which produces a detectable response that is a function of the presence of the analyte in the sample. Such homogeneous specific binding assay systems can include a label which participates in an enzymatic reaction. More particularly, one preferred format of the homogeneous specific binding assay reagent and system include an antibody which binds the analyte, a conjugate of the analyte or a binding analog thereof and a label, and a detectant system which interacts with the label to produce a detectable response that is different when the label conjugate is bound by the antibody compared to when it is not so bound. Any of the various label systems which are known to be suitable for use in homogeneous immunoassays are applicable for use in the present device and method.

The analytical element consists of a single integral matrix combined with a composition as described above. The single layer is all that is required for performance of a complete analysis. This provides freedom from the requirement of multiple distinct spreading reagent, reflection and other layers. The elements of the present invention can be made by suitable techniques such as incorporating solutions of the compositions into a preformed sintered polymeric matrix of the particles and allowing the combination so prepared to set or solidify, in the matrix. Although not required, the integral analytical elements can be provided on a support for ease of handling to form a device of the invention. Preferred supports include those of polystyrene, Mylar or similar plastics. The support can be opaque, translucent or transparent to light or other energy.

As previously noted, many of the reagent systems which can be used provide, or can be readily adapted to provide, a detectable response, such as a color change, related to the presence or amount of the analyte under assay in the liquid sample. The element of the invention provides enhanced electro-magnetic radiation signals such as a change in light reflectance in the visible spectrum, thereby producing a visible color change, or a change in light reflectance outside the visible range such as in the ultraviolet range or infrared range. The detectable response is one which can be observed through the senses directly or by use of ancillary detection means, such as a spectrophotometer or other sensing means. After the analytical result is obtained as a detectable change, it is measured, usually by passing the test element through a field in which suitable apparatus for reflectance measurement is provided. Such apparatus serves to direct a beam of energy, such as light, which is then reflected from the element back to a detector. Generally, electro-magnetic radiation in the range of from about 200 to about 900 nm has been found useful for such measurements.

The following section describes working examples using porous plastics as matrices for the "dry chemical" reactions. The porous plastics in various pore sizes and thicknesses were obtained from Porex Technologies, Fairburn, GA. The plastic polymer was high density polyethylene (HDPE), unless otherwise stated. The material was rendered hydrophilic by treating with 0.5 percent w/v Span® 20 ICI (United States, Inc., Wilmington DE) in methanol, and one side was heat sealed to prevent leakage whereever necessary. Circular disks of 12,7 mm (1/2 inch) diameter were cut out from sheets of HDPE, individually spotted with the specified volume of a reagent solution, and were either dried under ambient conditions or freeze dried. All measurements were done by spotting the circular disks with a specified sample volume and reading the percent reflectance with an InfraAlyzer 300 reflectance spectrometer (Technicon Instruments Corporation, Tarrytown, NY) which was modified to cover the spectral range between 340 and 700 nm. The reflectance (R) measurements were transformed to a linear function of analyte concentration $K/S = (1 - R)^2/2R$, where K is the absorption coefficient and S is the scattering coefficient.

EXAMPLE 1

SENSITIVITY COMPARISON FOR VARIOUS MATERIALS

Various support materials were tested for the detection of a low molar absorptivity (E) species NADH (E equals approximately $6 \times 10^3$), which is the indicator species in several clinical assays involving low concentration of analytes, such as alanine aminotransferase, aspartate amino-transferase, creatinine, and lactate dehydrogenase.

The support materials chosen for comparison were barium sulfate reflective coating (150 µm thick), Whatman Paper Nos. 41 and 42, 1,6 mm (1/16 - inch) thick porous Kynar® having a pore size of 25 micrometers, and 1,6 mm (1/16-inch) thick porous HDPE having pore sizes of 10, 35, 70, and 120 µm, respectively.

The series of test solutions used contained NADH at $1 \times 10^{-6}$, $5 \times 10^{-6}$, $1 \times 10^{-5}$, $5 \times 10^{-5}$, $1 \times 10^{-4}$, $5 \times 10^{-4}$, and $1 \times 10^{-3}$ mole/liter in 0.1 M tris buffer (pH 7.8).

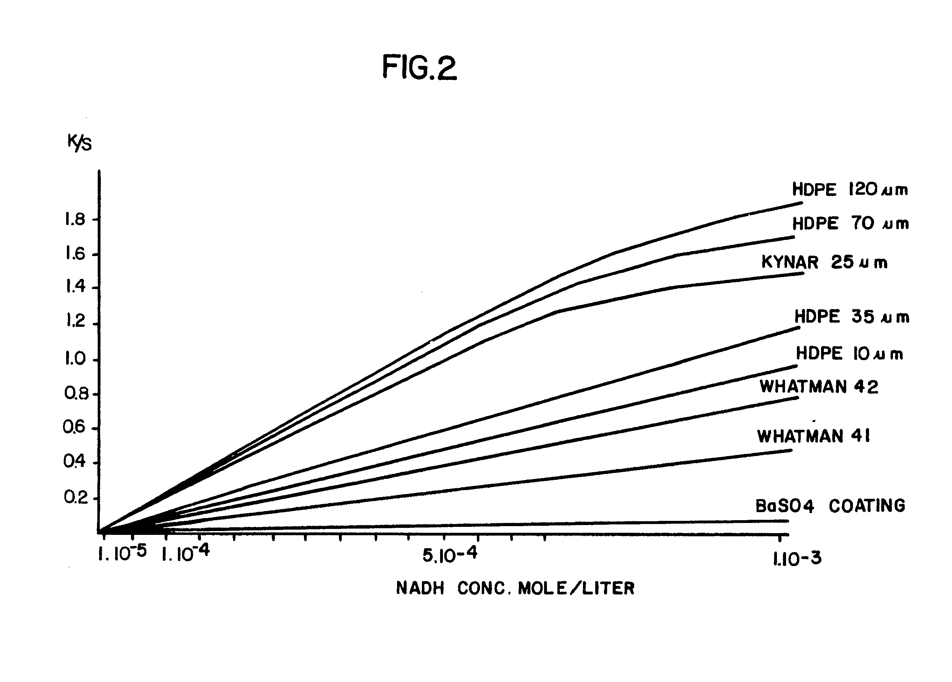

A 70 $\mu$l volume of sample was spotted on a 12,7 mm (1/2 inch) diameter disk cf each material, and the responses observed at 340 nm are summarized in Fig. 2.

The sensitivity, as determined by the slope of the plot of K/S versus concentration, was found to be highest for Porex® porous HDPE and Kynar®, yielding a detection limit of $1 \times 10^{-5}$ M NADH, which is suitable for all NADH-dependent clinical tests. Whatman paper materials and barium sulfate reflective coating gave less sensitive response curves and thus are unsuitable for many of the assays mentioned above. For a given material, for example porous HDPE, the sensitivity increases with increase in pore sizes. For the same or comparable pore size, the material that has the lower refractive index (RI), for instance Kynar® (RI 1.42) gives higher sensitivity than HDPE (RI 1.55).

## EXAMPLE 2

### ALANINE AMINOTRANSFERASE (ALT) ASSAY WITH POROUS PLASTIC MATRIX

To each disk of the porous plastic HDPE (35 $\mu$m pore size), 0.8 mm (1/32 inch) thick, 30 $\mu$l of a reagent mixture containing 100 mmol/liter tris buffer, pH 7.8, 5.0 mmol/liter EDTA, 100 mmol/liter alanine, 1.5 mmol/liter NADH, 1 mmol/liter pyridoxal phosphate, 10 mmol/liter 2-oxoglutarate sodium salt, and 11,100 IU/liter lactate dehydrogenase were deposited, and the disks were allowed to dry at room temperature for two hours while protected from any direct light source.

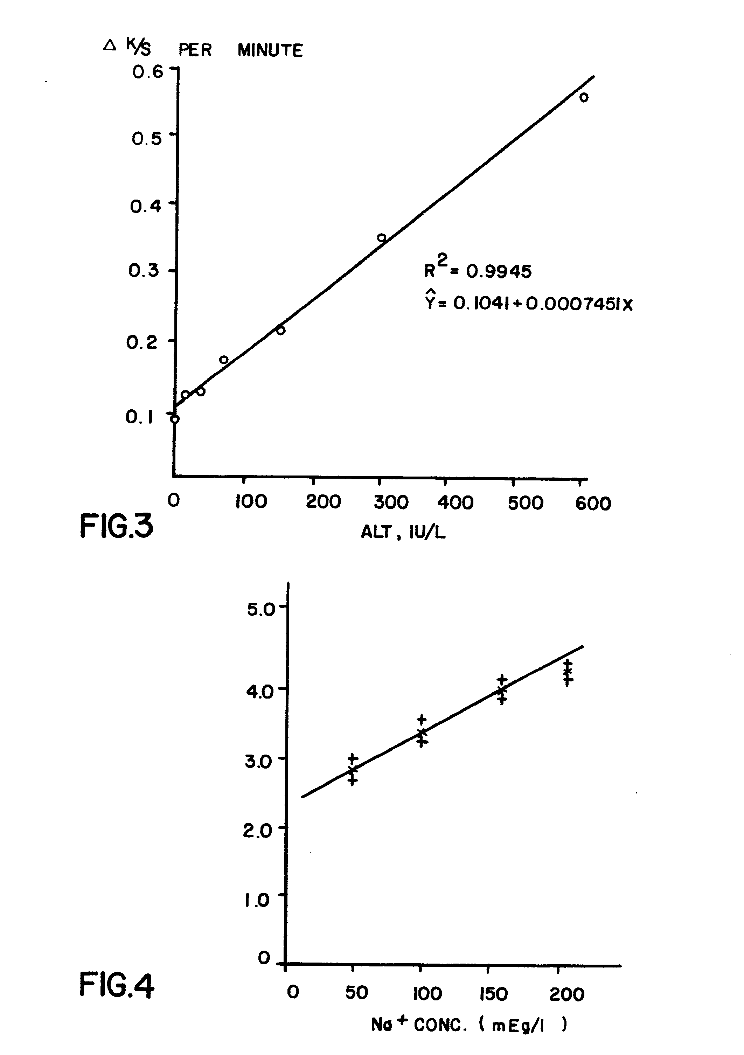

These disks were tested with 30 $\mu$l samples containing alanine aminotransferase (ALT). The concentration of ALT was varied to cover the clinical range, i.e., 0 to 600 IU/liter. The reflectance was recorded every 7.5 seconds for three to five minutes. The change in K/S response per minute was calculated at 30 seconds after the sample application to the disk. The plot of change in K/S response per minute versus ALT concentration is linear as shown in Fig. 3, indicating the efficacy of the analytical element.

The following Examples, 3 and 4, illustrate the application of porous polymeric matrix to solvent extraction technique which involves the extraction of the analyte species (sodium, potassium) into a low vapor pressure solvent (tricresylphosphate) containing reagents to cause a color change (chromogenic crown ether) upon reaction with the extracted analyte species. Further details relating to the solvent extraction methodology can be found in our EP-A-0 141 647.

## EXAMPLE 3

### SODIUM ASSAY

A porous plastic disk of ultra-high molecular weight polyethylene (10 $\mu$m, 1,6 mm (1/16inch) thick), was spotted with 100 $\mu$l of a reagent mixture containing 0.75 g tricresylphosphate, 0.25 g Span® 20 ICI, 50 mg 2-hydroxy-5-(4-nitrophenylazo)-phenyl)-oxymethyl-15-crown-5, 0.48 g cellulose acetate, 7 ml methylene chloride, 2 ml absolute ethanol, 1.5 ml isopropanol, and was allowed to dry at room temperature for one hour in a fume hood. An 80 $\mu$l volume of 0.3 M diethanolamine buffer (pH 90) was spotted on the disk with was again dried. Another crown ether which is also applicable is 4' -(2",4" -dinitro-6''-trifluoromethylphenyl) amino-benzo-15-crown-5.

The disks were tested with 80 $\mu$l samples containing various concentrations of sodium chloride in the clinical range from 50 meq/l to 200 meq/l. The diffused reflective signals, after five minutes incubation, were measured. The plot of K/S versus sodium concentration is linear, as shown in Fig. 4.

## EXAMPLE 4

### POTASSIUM ASSAY

To each disk, (HDPE, 35 $\mu$m, 0,8 mm (1/32-inch) thick), 40 $\mu$l of a reagent mixture containing 0.75 g tricresylphosphate, 0.25 Span® 20 ICI, 0.48 g cellulose acetate, 5 mg 4'-(2" ,4"-dinitro-6''-trifluoromethylphenyl)aminobenzo-18-crown-6, 7 ml methylene chloride, 2 ml absolute ethanol, and 1.5 ml isopropanol were deposited, and the disks were allowed to dry at room temperature for one hour. A 30 $\mu$l volume of 0.3 M diethanolamine buffer (pH 9,0) was spotted on disk which was again dried. Another crown ether which can also be used is 4'-picrylaminobenzo-18-crown-6.

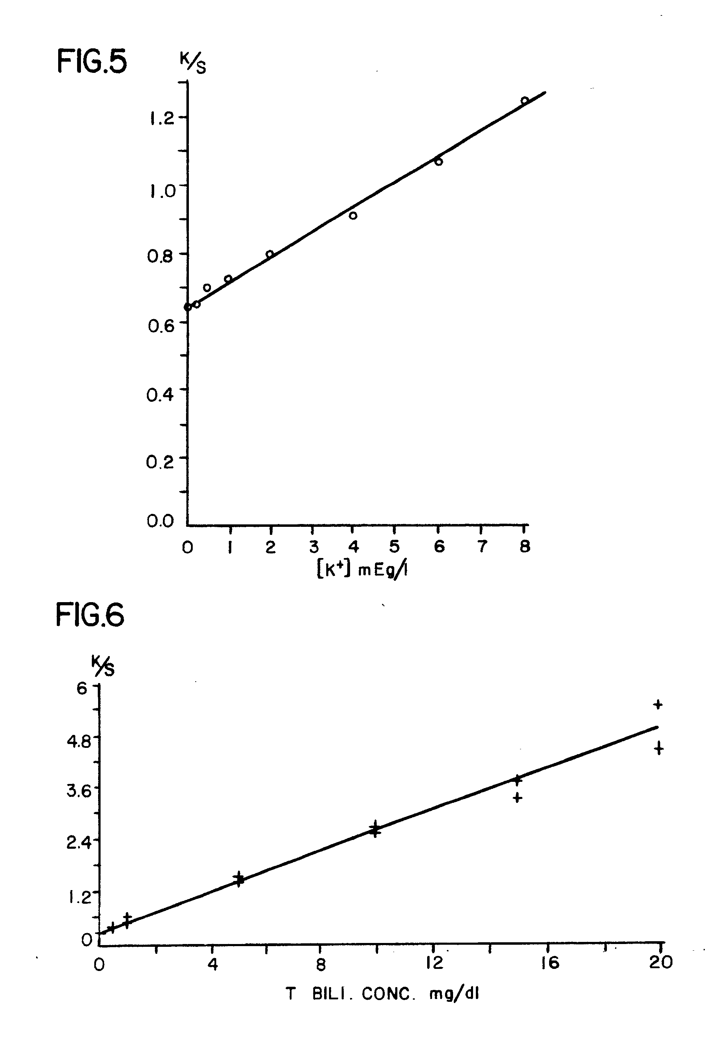

The disks were tested with 30 $\mu$l samples containing various concentrations of potassium chloride in the clinical range from 2 meq/l to 8 meq/l. The diffused reflective signals after five minutes incubation were measured at 450 nm. The plot of K/S versus potassium concentration is linear, as shown in Fig. 5.

The next two examples, 5 and 6, demonstrate the excellent sensitivity afforded by porous plastic for the assay of low concentration analytes in blood, such as bilirubin and uric acid, where the average normal concentrations are of the 5 order of 0.5 mg/dl and 7.5 mg/dl, respectively.

## EXAMPLE 5

BILIRUBIN ASSAY

The individual disk (HDPE, 70 $\mu$m, 1,6mm (1/16-inch) thick) was deposited with 100 $\mu$l of a reagent mixture containing 0.3 M diphylline, one percent v/v Triton® X-100, 79 mM diazotized sulfanilic acid, 2.26 M maleic acid, 0.76 M boric acid, and two percent (w/v) polyvinylpyrrolidone (MW 40,000) and was allowed to dry at room temperature for two hours while protected from any direct light source.

The disks were tested with 80 $\mu$l sample containing bilirubin at concentrations covering the clinical range of 0 to 20 mg/dl. This diffused reflective signals after five minutes incubation were measured at 540 nm. The plot of K/S versus bilirubin concentration is linear, as shown in Fig. 6. The slope of the plot, which is the measure of the sensitivity, indicates an excellent resolution of ± 0.1 mg/dl bilirubin.

EXAMPLE 6

URIC ACID ASSAY

Each disk of the porous plastic, HDPE (70 $\mu$m) 1,6 mm (1/16-inch) thick, was spotted with 100 $\mu$l of a reagent mixture containing 0.2 M phosphate, pH 8.5, 20 mM sodium azide, 1 mM EDTA, one percent (w/v) hydroxypropylmethyl cellulose, ten percent (w/v) lactose, 375,000 IU/liter peroxidase, 40,000 IU/liter uricase, 25 g/l BSA, 50 g/l 4-aminoantipyrine, and 75 g/l sodium p-hydroxybenzoate. The disks were allowed to dry at room temperature for two hours.

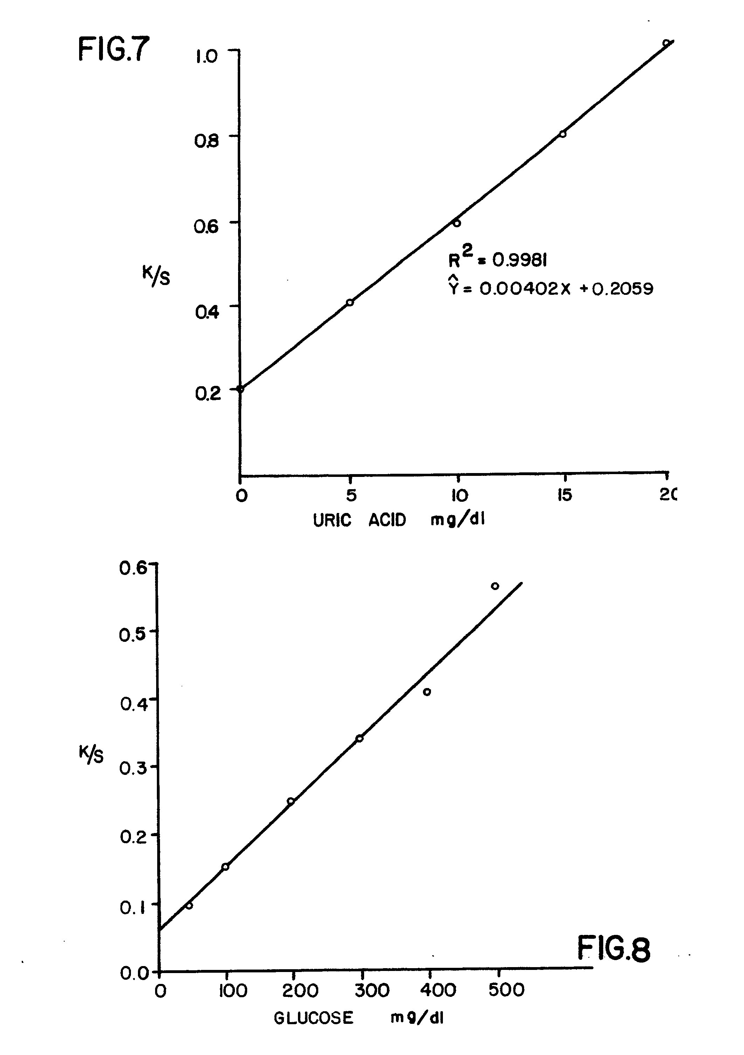

The disks were tested with 50 $\mu$l sample containing uric acid. Various concentrations of uric acid were used to cover the clinical range of 0 to 20 mg/dl. The measurements were done at 540 nm. The plot of K/S versus uric acid concentration is linear as shown in Figure 7, with good sensitivity to resolve ± 0.2 mg/dl uric acid.

EXAMPLE 7

GLUCOSE ASSAY

The application of porous plastics is not limited to the analysis of low concentration analytes only. The high degree of uniformity of porous plastics, coupled with the choice of various polymers and polymer blends with pigments like titanium oxide to modify the optical properties, allows them to be advantageously used for the assay of high concentration/high molar absorptivity species as well. In this example, the assay of a high concentration analyte, such as glucose (average normal concentration of 90 to 100 mg/dl), is achieved simply by making measurements at a wavelength where the light absorption is less than maximum.

To each disk of the porous plastic, HDPE (35 $\mu$m) 0,8 mm (1/32-inch) thick, 40 $\mu$l of a reagent mixture containing 3,000 U glucose oxidase, 3,000 IU peroxidase, 100 mg BSA, 200 mg 4-aminoantipyrine, 600 mg sodium p-hydroxybenzoate, 40 mg polyvinylpyrrolidone (MW 40,000), 0.04 m moles sodium azide, 0.002 m moles EDTA in 2 ml 0.2 M phosphate buffer, pH 7.0, were deposited, and the disks were allowed to dry at room temperature for two hours.

These disks were tested with 30 $\mu$l samples containing glucose at various concentrations covering the clinical range of 0 to 600 mg/dl. The measurements were done at 650 nm. The reflectance response of each sample after five minutes incubation were recorded. A plot of K/S versus glucose concentration was found to be linear, as shown in Fig. 8. Since the concentration of glucose in clinical serum samples is relatively high, measurements were made at 650 nm instead of at the absorption maximum wavelength of 500 nm.

**Claims**

1. A single layer analytical element for determining an analyte in a fluid sample, which consists of a dimensionally stable, uniformly porous, diffusely reflective single layer (20) formed of a polymeric non-fibrous matrix and, dispersed throughout said matrix, at least one analyte-responsive substance which effects a detectable response to said analyte.

2. An element according to claim 1, wherein the matrix is of a high density polyethylene, ultra-high molecular weight polyethylene, polypropylene, polyvinylidene fluoride, polytetrafluoroethylene, nylon®, polyvinylchloride, polyester, polysulfone or any blend of two or more thereof.

3. An element according to claim 2, wherein the matrix is coated with a hydrophilic surfactant.

4. An element according to claim 3, wherein the surfactant is selected from polyoxyethylene ethers of aliphatic alcohols, polyoxyethylene derivatives of sorbitol anhydride fatty acid partial esters, and alkylaryl polyethers of alcohols, sulfonates or sulfates.

5. An element according to any of claims 1 to 4, wherein (a) the analyte is alanine aminotransferase and the analyte responsive substances comprise alanine, NADH, pyridoxal phosphate, 2-oxoglutarate sodium salt and lactate dehydrogenase; or (b) the analyte is sodium and the analyte-responsive substance comprises a compound selected from 2-hydroxy-5-(4-nitrophenylazo)-phenlyoxy-methyl-15-crown-5 and 4'-(2", 4"-dinitro-6"-trifluoromethylphenyl) aminobenzo-15-crown-5; or the analyte is potassium and the analyte-responsive substance comprises a compound selected from 4'-(2",4"-dinitro-6"-trifluromethylphenyl) aminobenzo-18-crown-6and 4'-picrylaminobenzo-18-crown-6; or the analyte is bilirubin and the at least one analyte-responsive substance includes a diazo compound and a displacing agent.

6. An element according to claim 5, wherein the diazo compound is diazo sulfanilic acid, and the displacing agent is diphylline, caffeine or dimethylsulfoxide.

7. An element according to any of claims 1 to 4, wherein the analyte-responsive substance includes a first component which is responsive to the analyte to form a reaction product, and a second component responsive to the reaction product to effect a detectable change.

8. An element according to claim 7, wherein the first component is reactive with the analyte to produce hydrogen peroxide and the second component includes a peroxidatively active substance and a redox indicator and is reactive with hydrogen peroxide to produce a detectable response.

9. An element according to claim 8, wherein (a) the analyte is cholesterol and the first component includes cholesterol oxidase and cholesterol ester hydrolase; or (b) the analyte is uric acid and the first component includes uricase; or (c) the analyte is glucose and the first component includes glucose oxidase.

10. An element according to any of claims 1 to 4, wherein the analyte-responsive substance comprises reagents for a homogeneous specific binding assay system which produces a detectable response that is a function of the presence of the analyte in the sample.

11. An element according to claim 10, wherein (a) said homogeneous specific binding assay system includes a label which participates in an enzymatic reaction; or (b) said homogeneous specific binding assay system comprises an anti which binds said analyte, a conjugate of said analyte or a binding analog thereof and a label, and a detectant system which interacts with said label to produce a detectable response that is different when the label conjugate is bound by the antibody compared to when it is not so bound.

12. An element according to any of claims 1 to 11, which further comprises irregularly shaped, reflective, inorganic particles.

13. An element according to claim 12 wherein said particles are selected from basic lead hydroxide, basic lead carbonate, titanium dioxide, zinc oxide, magnesium oxide, barium sulfate, calcium sulfate, strontium sulfate, silica, calcite, silicates, aluminosilicates, zinc sulfide, diatomaceous earth, clay or any mixture of two or more thereof.

14. An analytical device which comprises an analytical element according to any of claims 1 to 13 mounted on an inert carrier.

15. A method of determining an analyte in a fluid sample, which method comprises contacting the sample suspected to contain the analyte with an element as claimed in any of claims 1 to 13 or a device as claimed in claim 14 appropriate to said analyte, and observing for a detectable response.

**Revendications**

1. Elément analytique monocouche pour déterminer un analyte dans un échantillon fluide, qui est composé d'une couche unique (20), de dimensions stables, uniformément poreuse et à réflexion diffuse, formée d'une matrice polymère non fibreuse et d'au moins une substance sensible à l'analyte, dispersée à travers ladite matrice, qui produit une réponse décelable à l'analyte en question.

2. Elément selon la revendication 1, caractérisé en ce que la matrice est composée d'un polyéthylène de densité élevée, d'un polyéthylène de poids moléculaire très élevé, de polypropylène, de fluorure de polyvinylidène, de polytétrafluoroéthylène, de Nylon®, de chlorure de polyvinyle, de polyester, de polysulfone ou de n'importe quel mélange de deux ou plusieurs de ces composés.

3. Elément selon la revendication 2, caractérisé en ce que la matrice est enduite d'un agent tensio-actif hydrophile.

4. Elément selon la revendication 3, caractérisé en ce qu'on choisit l'agent tensio-actif parmi des éthers polyoxyéthyléniques d'alcools aliphatiques, des dérivés polyoxyéthyléniques d'esters partiels d'acides gras d'anhydrides de sorbitol, et des polyéthers alkylaryliques d'alcools, des sulfonates ou des sulfates.

5. Elément selon l'une quelconque des revendications 1 à 4, caractérisé en ce que (a) l'analyte est l'alanine aminotransférase et les substances sensibles à l'analyte comprennent l'alanine, le NADH, le phosphate de pyridoxal, le sel de sodium de 2-oxoglutarate et la lacticodéshydrogénase ; ou (b) l'analyte est le sodium et la substance sensible à l'analyte comprend un composé choisi parmi la 2-hydroxy)-5-(4-nitrophénylazo)-phényloxy-méthyl-15-couronne-5et la 4'-(2", 4"-dinitro-6"-trifluorométhylphényl)aminobenzo-15-couronne-5 ; ou l'analyte est le potassium et la substance sensible à l'analyte comprend un composé choisi parmi la 4'-(2", 4"-dinitro-6"-trifluorométhylphényl)aminobenzo-18-couronne-6 et la 4'-picrylaminobenzo-18-couronne-6 ; ou l'analyte est la bilirubine et la substance minimale sensible à l'analyte comprend un composé diazo et un agent de déplacement.

6. Elément selon la revendication 5, caractérisé en ce que le composé diazo est un acide diazosulfanilique et l'agent de déplacement est la diphylline, la caféine ou le diméthylsulfoxyde.

7. Elément selon l'une quelconque des revendications 1 à 4, caractérisé en ce que la substance sensible à l'analyte comprend un premier composant qui est sensible à l'analyte, pour former un produit réactionnel, et un deuxième composant sensible au produit réactionnel, pour produire un changement décelable.

8. Elément selon la revendication 7, caractérisé en ce que le premier composant réagit avec l'analyte, pour produire du peroxyde d'hydrogène, et le deuxième composant comprend une substance peroxydiquement active et un indicateur d'oxydoréduction, et il réagit avec le peroxyde d'hydrogène, pour produire une réponse décelable.

9. Elément selon la revendication 8, caractérisé en ce que (a) l'analyte est le cholestérol et le premier composant comprend la cholestéroloxydase et la cholestérol ester-hydrolase ; ou (b) l'analyte est l'acide urique et le premier composant comprend l'uricase ; ou (c) l'analyte est le glucose et le premier composant comprend la glucose-oxydase.

10. Elément selon l'une quelconque des revendications 1 à 4, caractérisé en ce que la substance sensible à l'analyte comprend des réactifs d'un système d'essai homogène de liaison spécifique qui produit une réponse décelable qui dépend de la présence de l'analyte dans l'échantillon.

11. Elément selon la revendication 10, caractérisé en ce que (a) ledit système d'essai homogène de liaison spécifique comprend un marqueur qui participe à une réaction enzymatique ; ou (b) ledit système d'essai homogène de liaison spécifique comprend un anticorps qui fixe ledit analyte, un conjugué dudit analyte ou une liaison analogue de celui-ci, et un système de détection qui agit mutuellement avec ledit marqueur pour produire une réponse décelable qui est différente selon que le conjugué de marqueur est fixé par l'anticorps ou qu'il n'est pas fixé de cette manière.

12. Elément selon l'une quelconque des revendications 1 à 11, caractérisé en ce qu'il comprend en outre des particules de forme irrégulière, réfléchissantes et inorganiques.

13. Elément selon la revendication 12, caractérisé en ce qu'on choisit lesdites particules parmi l'hydroxyde basique de plomb, le carbonate basique de plomb, le dioxyde de titane, l'oxyde de zinc, l'oxyde de magnésium, le sulfate de baryum, le sulfate de calcium, le sulfate de strontium, la silice, des silicates, des aluminosilicates, le sulfure de zinc, la diatomite, l'argile ou n'importe quel mélange de deux ou plus de ces composés.

14. Dispositif analytique, caractérisé en ce qu'il comprend un élément analytique selon l'une quelconque des revendications 1 à 13, monté sur un support inerte.

15. Procédé de détermination d'un analyte dans un échantillon fluide, caractérisé en ce qu'il comprend la mise en contact de l'échantillon sensé contenir l'analyte avec un élément selon l'une des revendications 1 à 13, ou un dispositif selon la revendication 14, et l'observation d'une réponse décelable.

**Patentansprüche**

1. Einschichtiges analytisches Element zur Bestimmung einer zu analysierenden Substanz in einer Fluidprobe, das aus einer maßhaltigen, gleichmäßig porösen, diffus reflektierenden Einzelschicht (20) besteht, die aus einer polymeren, nicht faserförmigen Matrix und mindestens einer auf die zu analysierende Substanz ansprechenden, über und durch die Matrix verteilten Substanz gebildet ist, die eine erfaßbare Reaktion auf die zu analysierende Substanz bewirkt.

2. Element gemäß Anspruch 1, worin die Matrix aus einem hochdichten Polyethylen, ultrahochmolekularem Polyethylen, Polypropylen, Polyvinylidenfluorid, Polytetrafluorethylen, Nylon®, Polyvinylchlorid, Polyester, Polysulfon oder einem Gemisch zweier oder mehrerer dieser Substanzen besteht.

3. Element gemäß Anspruch 2, worin die Matrix mit einem hydrophilen oberflächenaktiven Mittel überzogen ist.

4. Element gemäß Anspruch 3, worin das oberflächenaktive Mittel aus Polyoxyethylenethern von aliphatischen Alkoholen, Polyoxyethylenderivaten von Fettsäurepartialestern von Sorbitanhydriden und Alkylarylpolyethern von Alkoholen, Sulfonaten oder Sulfaten ausgewählt ist.

5. Element gemäß einem der Ansprüche 1 bis 4, worin (a) die zu analysierende Substanz Alaninaminotransferase ist und die auf die zu analysierende Substanz ansprechende Substanz Alanin, NADH, Pyrodoxalphosphat, das Natriumsalz von 2-Oxoglutarat und Lactatdehydrogenase enthält oder (b) die zu analysierende Substanz Natrium ist und die auf die zu analysierende Substanz ansprechende Substanz eine Verbindung enthält, die aus 2-Hydroxy-5-(4-nitrophenylazo)phenyloxymethyl-[15] krone-5 und 4'-(2'',4''-Dinitro-6''-trifluoromethylphenyl)-aminobenzo-[15] krone-5 ausgewählt ist, oder die zu analysierende Substanz Kalium ist und die auf die zu analysierende Substanz ansprechende Substanz eine Verbindung enthält, die aus 4'-(2'',4''-Dinitro-6''-trifluoromethylphenyl)-aminobenzo[18] krone-6 und 4'-Picrylaminobenzo-[18] krone-6 ausgewählt ist, oder die zu analysierende Substanz Bilirubin ist und die mindestens eine auf die zu analysierende Substanz ansprechende Substanz eine Diazoverbindung und ein Verdrängungsmittel enthält.

6. Element gemäß Anspruch 5, worin die Diazoverbindung Diazosulfanilsäure und das Verdrängungsmittel Diphyllin, Koffein oder Dimethylsulfoxid ist.

7. Element gemäß einem der Ansprüche 1 bis 4, worin die auf die zu analysierende Substanz ansprechende Substanz eine erste Komponente enthält, die auf die zu analysierende Substanz unter Ausbildung eines Reaktionsproduktes anspricht, sowie eine zweite Komponente, die auf das Reaktionsprodukt unter Bewirkung einer erfaßbaren Veränderung anspricht.

8. Element gemäß Anspruch 7, worin die erste Komponente mit der zu analysierenden Substanz unter Bildung von Wasserstoffperoxid reagieren kann und die zweite Komponente eine peroxidativ aktive Substanz und einen Redoxindikator enthält und mit Wasserstoffperoxid unter Bewirkung eines erfaßbaren Ansprechens reagieren kann.

9. Element gemäß Anspruch 8, worin (a) die zu analysierende Substanz Cholesterin ist und die erste Komponente Cholesterinoxidase und Cholesterinesterhydrolase enthält oder (b) die zu analysierende Substanz Harnsäure ist und die erste Komponente Uricase enthält oder (c) die zu analysierende Substanz Glucose ist und die erste Komponente Glucoseoxidase enthält.

10. Element gemäß einem der Ansprüche 1 bis 4, worin die auf die zu analysierende Substanz ansprechende Substanz Reagenzien für ein homogenes Assaysystem durch spezifische Bindung enthält, das ein erfaßbares Ansprechen als Funktion der Anwesenheit der zu analysierenden Substanz in der Probe hervorruft.

11. Element gemäß Anspruch 10, worin (a) das homogene Assaysystem durch spezifische Bindung eine Markierung enthält, die an einer enzymatischen Umsetzung teilnimmt, oder (b) das homogene Assaysystem durch spezifische Bindung einen Antikörper, der sich an die zu analysierende Substanz, ein Konjugat der zu analysierenden Substanz oder ein bindendes Analogon davon und eine Markierung bindet, sowie ein Erfassungssystem umfaßt, das mit der Markierung in Wechselwirkung tritt, um ein erfaßbares Ansprechen zu erzeugen, das anders ist, wenn das markierte Konjugat durch den Antikörper gebunden ist, als dann, wenn es nicht durch den Antikörper gebunden ist.

12. Element gemäß einem der Ansprüche 1 bis 11, das außerdem unregelmäßig geformte reflektierende, anorganische Teilchen enthält.

13. Element gemäß Anspruch 12, worin die Teilchen aus basischem Bleihydroxid, basischem Bleicarbonat, Titandioxid, Zinkoxid, Magnesiumoxid, Bariumsulfat, Calciumsulfat, Strontiumsulfat, Siliciumdioxid, Calcit, Silikaten, Aluminosilikaten, Zinksulfid, Diatomenerde, Ton oder einem Gemisch zweier oder mehrerer Vertreter davon ausgewählt sind.

14. Analytische Vorrichtung, die ein analytisches Element gemäß einem der Ansprüche 1 bis 13 enthält, das auf einen inerten Träger aufgebracht ist.

15. Verfahren zur Bestimmung einer zu analysierenden Substanz in einer Fluidprobe, wobei man die Probe, von der man annimmt, daß sie zu analysierende Substanz enthält, mit einem Element gemäß einem der Ansprüche 1 bis 13 oder einer Vorrichtung gemäß Anspruch 14, die geeignet für die zu analysierende Substanz sind, in Berührung bringt und ein erfaßbares Ansprechen beobachtet.

# FIG.I

FIG.2

EP 0 169 055 B1

FIG.3

FIG.4

FIG.5

FIG.6

FIG.7

$R^2 = 0.9981$
$\hat{Y} = 0.00402X + 0.2059$

URIC ACID mg/dl

FIG.8

GLUCOSE mg/dl